# EUROPEAN PATENT SPECIFICATION

(11) **EP 1 443 996 B1**
(45) Date of publication and mention of the grant of the patent: **18.05.2011**
(21) Application number: 02778846.2
(22) Date of filing: 14.11.2002
(51) Int. Cl.: A61M 15/00

(54) **AEROSOLIZATION APPARATUS COMPRISING CONNECTABLE BODY AND ENDPIECE**
AEROSOL-VERNEBLER BESTEHEND AUS KOPPELBAREM HAUPT- UND ENDSTÜCK
APPAREIL DE PULVERISATION EN AEROSOL COMPRENANT UN CORPS ET UNE PARTIE D'EXTREMITE POUVANT ETRE RACCORDES

(30) Priority: 14.11.2001 US 336320 P
(43) Date of publication of application: 11.08.2004
(73) Proprietor: Novartis AG, 4002 Basel (CH); PH & T S.P.A., 20145 Milan (IT)
(72) Inventor: BURR, John, D., Redwood City, CA 94061 (US); WOOD, Jeff, R., Mountain View, CA 94040 (US); SMITH, Adrian, E., Belmont, CA 94002 (US); HOWARD, John, A., Palo Alto, CA 94306 (US); NICCOLAI, Fabrizio, I-20144 Milan (IT)
(74) Representative: Vossius & Partner
(86) International application number: PCT/US2002/036801
(87) International publication number: WO 2003/041776

(56) References cited:
- US-A- 5 301 666
- US-A- 5 447 151
- US-A- 5 989 217

## Description

### BACKGROUND

The need for effective therapeutic treatment of patients has resulted in the development of a variety of pharmaceutical formulation delivery techniques. One traditional technique involves the oral delivery of a pharmaceutical formulation in the form of a pill, capsule, elixir, or the like. However, oral delivery can in some cases be undesirable. For example, many pharmaceutical formulations may be degraded in the digestive tract before they can be effectively absorbed by the body. Inhaleable drug delivery, where an aerosolized pharmaceutical formulation is orally or nasally inhaled by a patient to deliver the formulation to the patient's respiratory tract, has proven to be a particularly effective and/or desirable alternative. For example, in one inhalation technique, a pharmaceutical formulation is delivered deep within a patient's lungs where it may be absorbed into the blood stream. Many types of inhalation devices exist including devices that aerosolize a dry powder, devices comprising a pharmaceutical formulation stored in or with an inhaleable propellant, devices which use a compressed gas to aerosolize a liquid pharmaceutical formulation, and similar devices.

US 5,989,217 discloses a medicine administering device for nasal cavities, comprising a capsule holding section formed with a capsule containing powder-state medicine. An air supply section is connected to the lower end of the capsule holding section and formed with an air supply passage which is in communication with the capsule accommodating chamber. A branched passage section is connected to the upper end of the capsule holding section and has an air flow passage in communication with the capsule accommodating chamber. The air flow passage is bifurcated to form first and second outlet passage portions which are arranged to form a generally Y-shape. First and second medicine passages are formed such that air from the branched passage section flows therethrough to carry the medicine in the capsule into right-side and left-side nasal cavities of a patient. The first and second passages are respectively in communication with the first and second outlet passages of the branched passage section. First and second spraying holes are respectively in communication with the first and second medicine passages, the medicine from the first medicine passage and the medicine from the second medicine passage being sprayed respectively from the first and second spraying holes. The first and second spraying holes respectively have first and second axes which are separate from each other by a distance ranging from 12 to 25 mm.

In one dry powder aerosolization technique, a capsule containing an inhaleable dry powder is loaded into a chamber in an aerosolization device. Within the chamber, the dry powder is at least partially emptied and dispersed to aerosolize the dry powder so that it may be inhaled by a patient. However, in conventional devices, the manner of accessing the chamber may often lead to device inconsistencies and/or failures. Also, the dry powder in the cavity can cause the access mechanism to become less effective at efficiently opening and closing.

Therefore, it is desirable to improve the manner of accessing an aerosolization device chamber. It is further desirable to access the chamber in a manner that reduces device inconsistencies and/or failures. It is still further desirable to access the cavity so that debris in the cavity will have reduced adverse affects on the functioning of the device.

### SUMMARY

The present invention satisfies these needs. In one aspect of the invention an aerosolization apparatus comprises a body and an endpiece, the body and endpiece being connectable to one another by a connection mechanism that prevents inadvertent disconnection of the parts.

In another aspect of the invention, an aerosolization apparatus comprises a body having an inlet, an endpiece having an outlet, the endpiece being connectable to the body to define a chamber, wherein the chamber is sized to receive a capsule containing a pharmaceutical formulation in a manner which allows the capsule to move within the chamber, a connection mechanism to provide selective connection of the endpiece to the body, wherein a rotational force between the endpiece and the body is needed to connect or disconnect the endpiece from the body, the rotational force being applied about an axis passing through the chamber, and a puncturing mechanism capable of providing an opening in the capsule, whereby when a user inhales, air enters into the chamber through the inlet so that the pharmaceutical formulation is aerosolized within the chamber and the aerosolized pharmaceutical formulation is delivered to the user through the outlet.

In another aspect of the invention, an aerosolization apparatus comprises a body having an inlet, an endpiece having an outlet, the endpiece being connectable to the body to define a chamber, wherein the chamber is sized to receive a capsule containing a pharmaceutical formulation in a manner which allows the capsule to move within the chamber, a connection mechanism to provide selective connection of the endpiece to the body, wherein the connection mechanism comprises engageable threads, and a puncturing mechanism capable of providing an opening in the capsule, whereby when a user inhales, air enters into the chamber through the inlet so that the pharmaceutical formulation is aerosolized within the chamber and the aerosolized pharmaceutical formulation is delivered to the user through the outlet.

In another aspect of the invention, an aerosolization apparatus comprises a body having an inlet, an endpiece having an outlet, the endpiece being connectable to the body to define a chamber, wherein the chamber is sized to receive a capsule containing a pharmaceutical formulation in a manner which allows the capsule to move within the chamber, a connection mechanism to provide selective connection of the endpiece to the body, wherein the connection mechanism comprises a protrusion that is receivable within a slot, the slot comprising a longitudinally extending portion and a transversely extending portion, and a puncturing mechanism capable of providing an opening in the capsule, whereby during inhalation air enters into the chamber through the inlet so that the pharmaceutical formulation is aerosolized within the chamber and the aerosolized pharmaceutical formulation is delivered to the user through the outlet.

In another aspect of the invention, a method of providing an aerosolized pharmaceutical formulation comprises providing a body and an endpiece, the endpiece being connectable to the body when a rotational force is applied thereto to define a chamber, the chamber being sized to receive a capsule containing a pharmaceutical formulation, wherein the rotation force is applied about an axis that passes through the chamber, and aerosolizing the pharmaceutical formulation when a user inhales by causing air to flow through an inlet in the body, within the chamber, and through an outlet in the endpiece to provide the aerosolized pharmaceutical formulation to the user.

In another aspect of the invention, a method of aerosolizing a pharmaceutical formulation comprises inserting a capsule containing a pharmaceutical formulation into a chamber in a body, rotating an endpiece relative to the body to connect the endpiece to the body, the rotation being about an axis passing through the chamber, before, during, or after inserting the capsule into the chamber, providing an opening in the capsule, and inhaling through an opening in the endpiece to cause air to flow into the chamber through an inlet in the body thereby aerosolizing the pharmaceutical formulation.

### DRAWINGS

These features, aspects, and advantages of the present invention will become better understood with regard to the following description, appended claims, and accompanying drawings which illustrate exemplary features of the invention. However, it is to be understood that each of the features can be used in the invention in general, not merely in the context of the particular drawings, and the invention includes any combination of these features, where:
Figure 1 is a schematic sectional side view of a version of an aerosolization device of the invention with an endpiece and body connected;
Figure 2 is a schematic sectional side view of the version of an aerosolization device of Figure 1 with the endpiece and body disconnected;
Figure 3 is a schematic sectional side view of a version of an aerosolization device in use;
Figure 4A is a schematic sectional side view of another version of an aerosolization device;
Figure 4B is a schematic prospective view of the aerosolization device of Figure 4A;
Figure 4C is a schematic section view along section A-A in Figure 4B;
Figures 5A through 5C are schematic views of versions of connection mechanisms for use with an aerosolization device;
Figure 6A is a schematic sectional side view of a portion of another version of an aerosolization device;
Figure 6B is a schematic end view of the body of the version of an aerosolization device of Figure 6A;
Figure 6C is a schematic sectional side view of the version of the aerosolization device of Figure 6A in a connected configuration;
Figures 7A and 7B are schematic sectional and schematic perspective views, respectively, of a portion of another version of an aerosolization device;
Figure 8 is a schematic perspective view of a portion of another version of an aerosolization device;
Figure 9 is a schematic side view of a body of a version of an aerosolization device;
Figure 10 is a schematic sectional side view of a body of another version of an aerosolization device; and
Figures 11A though 11M illustrate parts of a specific version of an aerosolization device.

### DESCRIPTION

The present invention relates to delivering an aerosolized pharmaceutical formulation to a patient. Although the process is illustrated in the context of aerosolizing a dry powder pharmaceutical formulation, the present invention can be used in other processes and should not be limited to the examples provided herein.

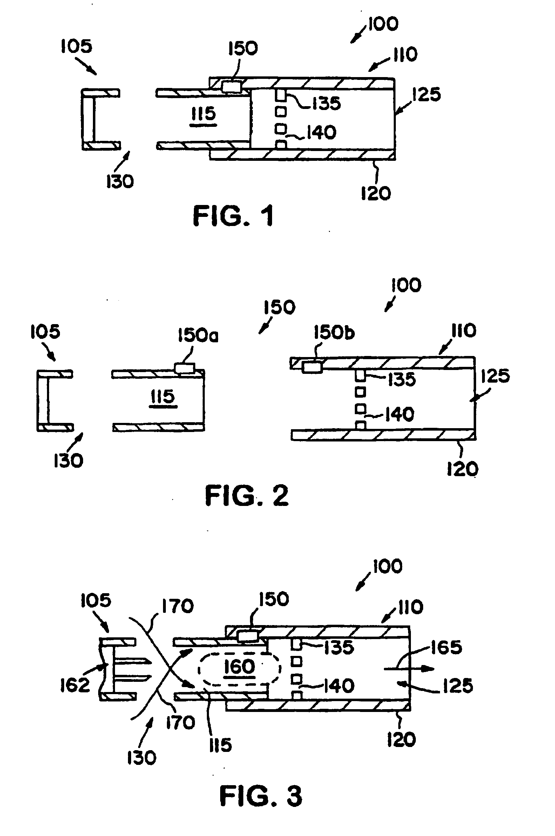

An aerosolization device **100** of the present invention is shown schematically in Figure 1. The aerosolization device **100** includes a body **105** and an endpiece **110** that may be attached to the body **105** to form a chamber **115** within the interior of the body **105** and the endpiece **110.** The endpiece **110** includes an end **120** defining an outlet **125.** The end **120** may be sized and shaped to be received in a user's mouth. Alternatively, the end **120** may be sized and shaped to be received in a nostril of a user or may sized and shaped to be received by a mask, a spacer chamber, a respirator circuit, or the like. The body includes one or more inlets **130** in communication with the chamber **115.** Together the inlets **130,** the chamber **115,** and the outlet **125** define an airway through the aerosolization device **100.** Accordingly, when a user contacts the endpiece **110** and inhales or otherwise creates a vacuum at the outlet **125,** a pharmaceutical formulation with the chamber **115** may be delivered to the user through the outlet **125.** In one version, the pharmaceutical formulation may be contained within a capsule that is positionable within the chamber **115,** the chamber **115** being sized to receive the capsule in a manner which allows the capsule to move within the chamber **115.** In this version, the endpiece **110** includes a perforated member **135** having one or more openings **140** therein. The perforated member **135** sufficiently blocks the chamber **115** to retain a capsule in the chamber **115,** while the openings **140** allow air and/or other material to pass to the outlet **125.** A connection mechanism **150** may be provided to allow the endpiece **110** to be attached to the body **105.**

In one version, as shown in Figure 2, the connection mechanism **150** may allow the body **105** and the endpiece **110** to be disconnected to allow for access to the chamber **115.** In this version, the endpiece **110** may be disconnected from the body **105** to allow a pharmaceutical formulation to be inserted into the chamber, for example by allowing a capsule to be inserted into the chamber **115.** In this version, the connection mechanism includes a body connection member **150a** that cooperates with an endpiece connection member **150b** to selectively connect and disconnect the endpiece **110** to the body **105.**

After a capsule **160** has been inserted into the chamber **115,** the endpiece **110** may again be attached to the body **105** to secure the capsule **160** within the chamber **115,** as shown in Figure 3. The capsule **160** is opened, for example by puncturing the capsule **160** prior to insertion or within the chamber **115,** such as by longitudinally advancing a sliding puncture mechanism **162.** When opened, the pharmaceutical formulation in the capsule is allowed to exit the capsule **160.** In one version, the pharmaceutical formulation is in a dry powder form and the flow of air through the airway causes the pharmaceutical formulation to be aerosolized. For example, as shown in Figure 3, a user may contact the endpiece **110** with his or her mouth and inhale, thereby drawing air through the outlet **125,** as shown by arrow **165.** This inhalation causes air to be taken in through the inlets **130,** as shown by arrows **170.** The air taken in causes the capsule **160** to agitate within the chamber **115.** The agitation causes the dry powder pharmaceutical formulation to leave the capsule **160** and become aerosolized in the airway. The aerosolized pharmaceutical formulation passes through the perforated member **135** and is delivered to the user where it may be inhaled to a position in the user's respiratory tract. In one particular embodiment, a plurality of inlets **130** may be designed to cause the inlet air **170** to swirl within the chamber, for example, by being at least partially tangentially oriented as described in U.S. Patent 4,995,385 and U.S. Patent 4,069,819.

In such an arrangement, the chamber **115** comprises a longitudinal axis that lies generally in the inhalation direction **165,** and the capsule **160** is insertable lengthwise into the chamber **115** so that the capsule's longitudinal axis may be parallel to the longitudinal axis of the chamber **115.** The swirling air flow then causes the capsule to rotate within the chamber **115** in a manner where the longitudinal axis of the capsule is remains at an angle less than 80 degrees, and preferably less than 45 degrees from the longitudinal axis of the chamber. In one version, this rotation is caused by the width of the chamber being less than the length of the capsule.

Often, a user will grasp the body **105** during use while inhaling through the endpiece **110.** It has been discovered that doing so may create a disconnection force in the inhalation direction **165** between the body **105** and the endpiece **110.** Accordingly, the connection mechanism **150** may be designed to prevent undesired disconnection of the endpiece **110** from the body **105** during use.

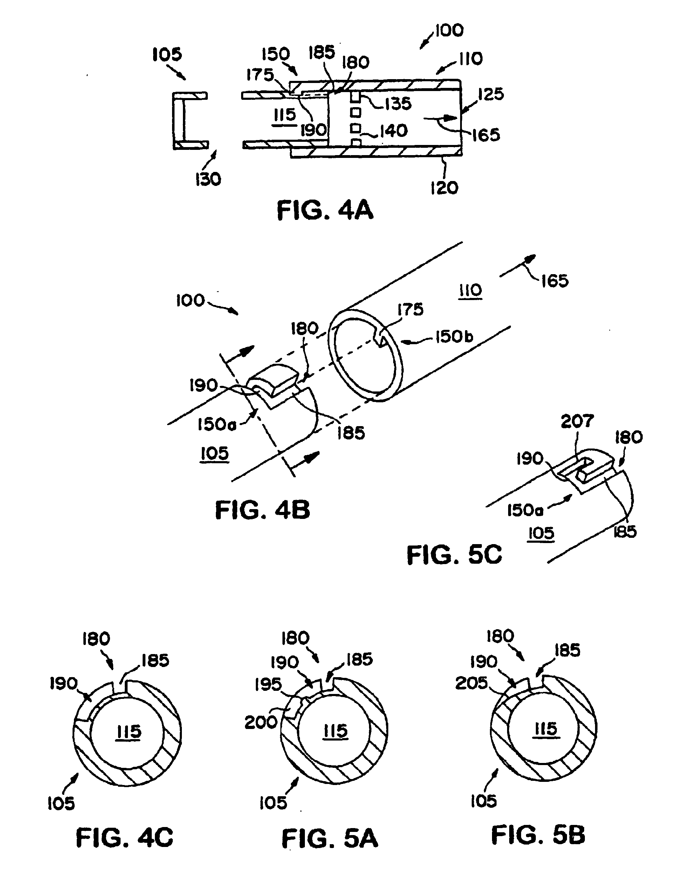

In one version, the connection mechanism **150** requires a force to be applied at least partially in a direction other than in an inhalation direction **165** in order to disconnect the endpiece **110** from the body **105.** Thus, in this version, the user's inadvertent forcing apart of the endpiece **110** and the body **105** during use does not generate a force in the direction required for disconnection. For example, the force required for disconnection may be a rotational force. In one particularly preferred version, the rotational force is a rotational force applied about an axis that passes through the chamber. For example, the rotational force may be applied about an axis that passes through the chamber and is parallel or coaxial with a longitudinal axis passing through the chamber. Such a rotational force is generally not generated by a user during inhalation making inadvertent disconnection more difficult. Examples of connection mechanisms of this type are schematically shown in Figures 4-10.

In the version of Figures 4A, 4B, and 4C the aerosolization device **100** comprises a connection mechanism **150** including a protrusion **175** on the endpiece **110** and a groove or slot **180** on the body **105.** Alternatively, the protrusion **175** may be provided on the body **105** and the slot **180** may be provided on the endpiece **110.** The protrusion **175** is insertable into the slot **180** for attachment of the endpiece **110** to the body **105.** For example, in the version shown, the slot **180** may comprise a longitudinally extending portion **185** and a transversely extending portion **190.** To connect the parts, the protrusion **175** is inserted into the longitudinally extending portion **185** until it is in a position in alignment with the transversely extending portion **190** at which time the endpiece **110** is twisted relative to the body **105** to cause the protrusion **175** to slide within the transversely extending portion **190.** When the protrusion **175** is within the transversely extending portion **190,** the walls of the transversely extending portion **190** prevent movement of the protrusion **175,** and thus movement of the endpiece **110** in the inhalation direction **165.** Accordingly, when the user inhales on the endpiece **110,** the endpiece **110** is prevented from disconnecting with the body **105** as a result of forces in the inhalation direction **165** alone. Figure 4C is a cross-section along line A-A of the transversely extending portion **190.** As shown, the transversely extending portion **190** extends partly around the circumference of the body **105.** Alternatively, the transversely extending portion **190** may extend completely around the body **105** or to a different circumferential position than the position shown.

The slot **180** may further be designed to help secure the protrusion **175** within the slot **180.** For example, as shown in Figures 5A, 5B, and 5C, the transversely extending portion **190** may include a member which serves to secure the protrusion **175** within the transversely extending portion **190.** In the version of Figure 5A, a projection **195** such as a bump is provided on the base of the transversely extending portion **190.** When sufficient rotational force is applied, the protrusion **175** may slide over the projection **195** to be positioned in a secured position **200** in the slot **180,** thereby providing a snap fit. To disconnect the parts, the endpiece **110** may be twisted in the opposite direction with sufficient force to cause the protrusion **175** to again slide past the projection **195.** Additionally or alternatively, one or more projections **195** may be provided on the side walls of the transversely extending portion **190.** In the version of Figure 5B, the depth of the base **205** of the transversely extending portion **190** gradually lessens. Accordingly, as the protrusion **175** is slid in the transversely extending portion **190,** the frictional forces increase and the protrusion **175** may be wedged into a secure position within the slot **180.** To disconnect this version, a sufficient force is applied to overcome the wedging of the protrusion **175** in the transversely extending portion **190.** Alternatively, the side walls of the transversely extending portion may be somewhat V-shaped to create the wedging effect. In the version shown in Figure 5C, the slot **180** comprises a second longitudinally extending portion **207** spaced from the longitudinally extending portion **185** and communicable therewith by the transversely extending portion **190.** When in the forward region of the second longitudinally extending portion **207,** the protrusion **175** is prevented from movement in the transverse direction and from movement in the inhalation direction **165.** Thus, force generated during inhalation does not cause disconnection. To disconnect, the endpiece is moved in a direction opposite to the inhalation direction **165.** A projection or a wedging surface, as discussed above, may be further provided in the second longitudinally extending portion **207** to further secure the protrusion **175.** Optionally, a biasing member, such as a compressed spring, may be positioned to bias the protrusion in the inhalation direction **165** when the protrusion is in the slot **180.** The biasing member will serve to secure the protrusion in the second longitudinally extending portion 207 until the user applies a force sufficient to overcome the bias.

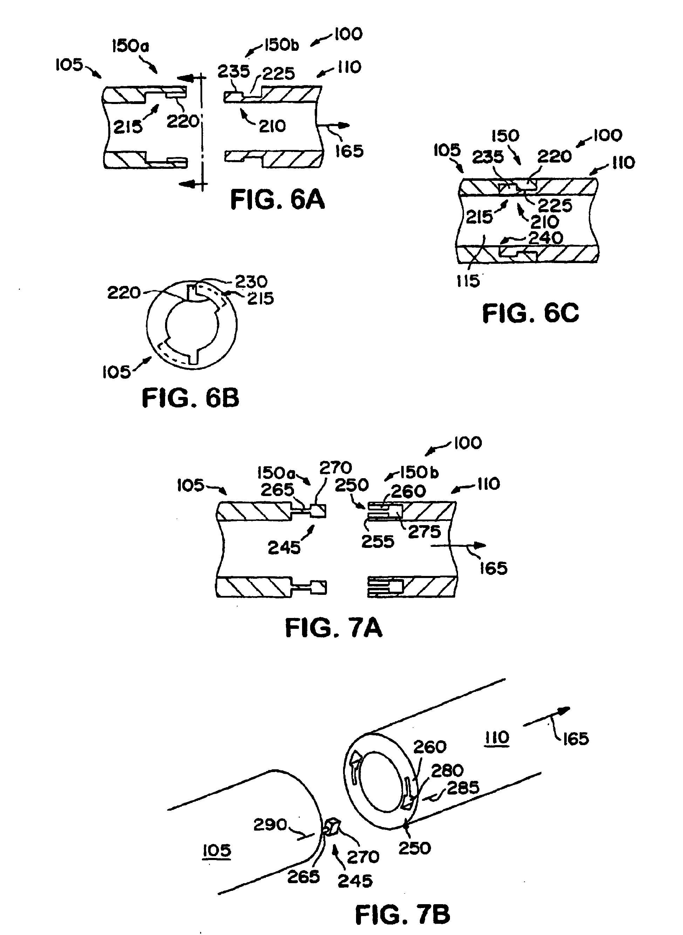

In another version, as shown for example in Figures 6A, 6B, and 6C, the aerosolization device **100** comprises a connection mechanism **150** with a longitudinally extending protrusion **210** that is receivable in an interior slot **215.** In the version shown, the longitudinal protrusion **210** is provided on the endpiece **110** and the interior slot **215** is provided on the body **105.** Alternatively, this may be reversed. The interior slot **215** includes a collar **220** which is receivable in a recess **225** on the protrusion **210** to prevent movement of the endpiece **110** in the inhalation direction **165** when the endpiece **110** is attached to the body **105,** as shown in the connected configuration shown in Figure 6C. A portion of the collar **220** is reduced in size or thinned to provide a longitudinal access **230** to the slot **215,** as best shown in Figure 6B which is an end view of the body **105** along line B-B. To attach the endpiece **110** to the body **105** in this version, the protrusion **210** is inserted into the longitudinal access **230** of the slot **215** until the end portion **235** extends beyond the collar **220.** The endpiece **110** is then twisted so that the end portion **235** is positioned behind the collar **220** and prevented from moving in the inhalation direction **165.** A snap fit projection or a sloped wedging surface, as discussed above, may be provided in the slot **215** to further secure the protrusion **210** within the slot **215.** Optionally, multiple protrusions and slots may be provided, as shown.

As can be seen in Figure 6C, this version of the aerosolization device **100** also provides a substantially smooth surface **240** within the chamber **115.** This smooth surface **240** may be advantageous in increasing the aerosolization space in the chamber **115** thereby creating more space in which a capsule may rattle. Additionally, the less discontinuous surface may provide more consistent rattling of the capsule and, thus, more consistent emptying of the capsule. Figures 7A and 7B show another version of a connection mechanism **150** that provides a smooth surface when in a connected configuration. In this version, a longitudinal protrusion **245** extends from the body **105** and is insertable into an opening **250** in the end surface **255** in the endpiece **110.** Alternatively, the protrusion **245** and opening **250** may be reversed. The opening **250** includes a collar **260** that may be engaged around or near a recess **265** on the protrusion **245** when in a connected configuration to prevent the endpiece **110** from moving relative to the body **105** in the inhalation direction **165.** To connect the parts, an end portion **270** of the protrusion **245** is inserted into the opening **250** at an enlarged area **280,** as best shown in Figure 7B. The parts are then rotated relative to one another so that the end portion **270** is secured within a cavity **275** beyond the collar **260.** The cavity **275** may have snap fit projections or wedging surfaces, as discussed above, to further secure the protrusion **245** within the opening **250.** Multiple protrusions **245** and openings **250** may be provided.

In the versions of Figures 4 through 7, indicia may be provided to aid the user when connecting or disconnecting the endpiece **110** to the body **105.** For example, as shown in the version of Figure 7B, a first marking **285** may be provided on the outer surface of the endpiece **110** and a second marking **290** may be provided on the outer surface of the body **105.** When the first marking **285** and the second marking **290** are aligned with one another, the two parts may be disconnected. Additionally or alternatively, markings may be provided to indicate to the user in which direction to twist the parts in order to connect or to disconnect the parts.

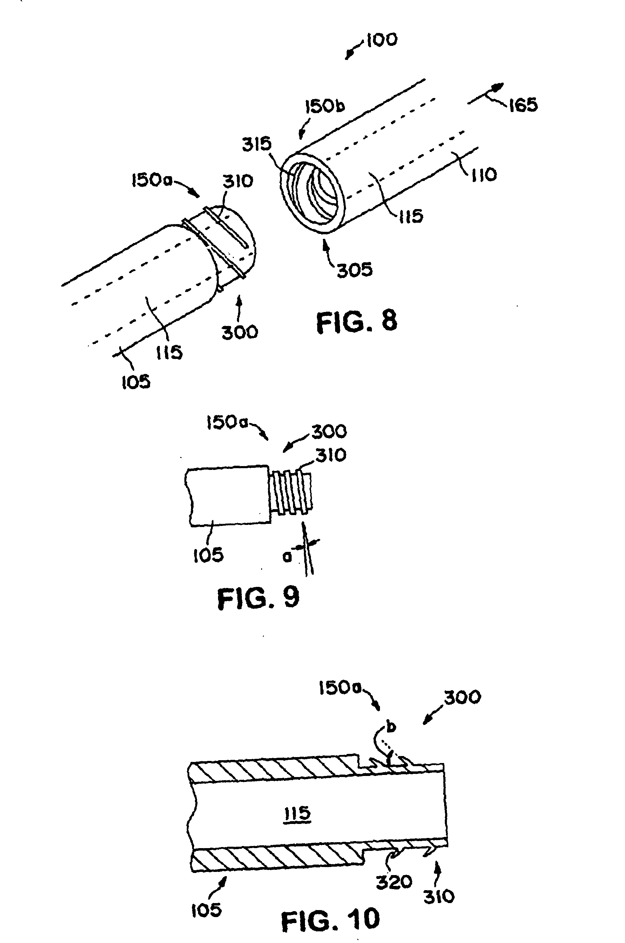

Another version of an aerosolization device **100** comprising a connection mechanism **150** that must at least partially be forced in a direction other than an inhalation direction **165** is shown in Figure 8. In this version, the body **105** includes a male portion **300** that is insertable into a female portion **305** on the endpiece **110.** On the male portion **300** are external threads **310** that may engage internal threads **315** on the endpiece **110.** Accordingly, the endpiece **110** may be attached to the body **105** by screwing the parts together. The threaded engagement prevents the endpiece **110** from disconnecting from the body **105** when a force in the inhalation direction **165** is applied. It has been discovered that this arrangement prevents disconnection of the parts during inhalation by a user. Alternatively, the threaded arrangement shown in Figure 8 may be switched so that the male portion **300** is on the endpiece **110** and the female portion **305** is on the body **105.** The threads may be standard helical threads. Alternatively, the threads may comprise a series of bumps and/or posts that mate in a screw-like manner.

The thread arrangement may be designed to further prevent disconnection of the endpiece **110** from the body **105** during use. For example, Figure 9 shows a version of a threaded portion having threads of high pitch. It has been determined that when the thread angle, a, is less than about 9 degrees, inhalation forces in the inhalation direction **165** are not sufficient to unscrew the parts. Accordingly, in one version, the threads have a thread angle of less than about 9 degrees, and more preferably less than about 7 degrees. Alternatively or additionally, the threads may be shaped to further prevent disconnection of the endpiece **110** and the body **105** when a force in the inhalation direction **165** is applied. For example, in the version shown in Figure 10, the threads have a recessed backside **320** to provide interlocking of the threads and thereby preventing stripping of the threads when stressed. In one version, the recess angle, b, is less than 90 degrees, more preferably less than about 75 degrees, and most preferably less than about 60 degrees. The mating threads on the opposing part are shaped to be received in the recessed backside **320.**

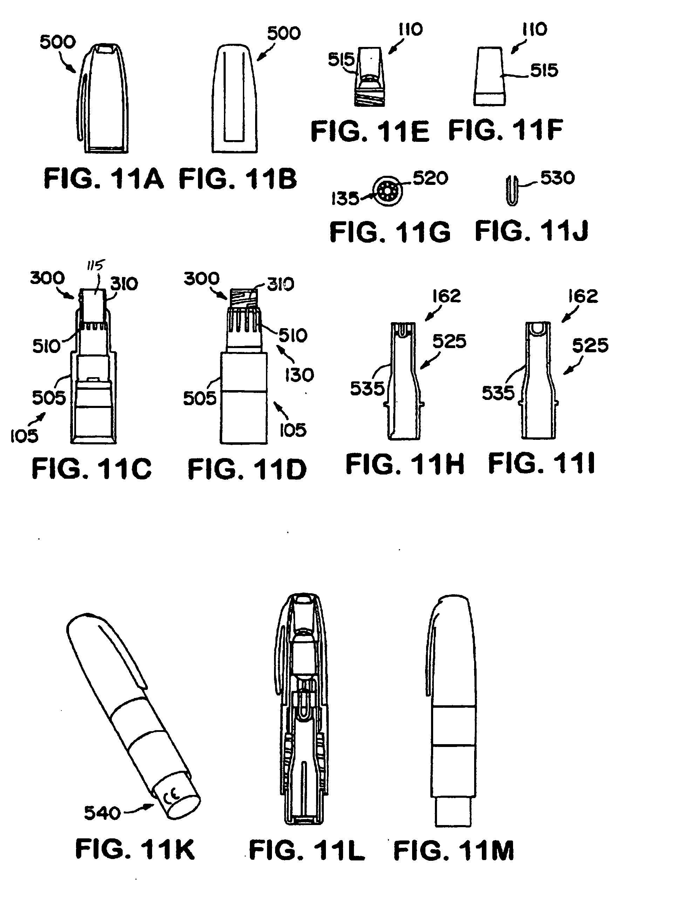

Figures 11A though 11M illustrate parts from a specific version of an aerosolization device **100.** Figures 11A and 11B show, respectively, a sectional view and a side view of a cap **500** that may be inserted over an endpiece **110.** Figures 11C and 11D show, respectively, a sectional view and a side view of a specific version **505** of a body **105.** The version includes a plurality of angled slots **510** that provide an inlet **130** into the chamber **115.** Figures 11E and 11F show, respectively, a sectional view and a side view of a version **515** of an endpiece **110.** The endpiece **110** may be connected and disconnect to the body **105** by rotational force. Figure 11G shows an end view of the endpiece **110** of Figure 11E showing an arcuate version **520** of a perforated member **135** within the endpiece **110.** Figures 11H and 11I show, respectively, a version **525** of a puncturing mechanism **162.** A U-shaped puncturing member **530,** as shown in Figure 11J, is seated in the end of a slidable member **535.** As shown in Figure 11K, a position **540** on the device may be used to provide a marking on the device. Figures 11L and 11K show, respectively, a sectional view and a side view of an assembled device according to the version of Figures 11A through 11J. To use the aerosolization device **100** of Figures 11A through 11J, a user takes an assembled device, as shown in Figure 11L, and removes the cap **500.** Then, the user twists the endpiece **515** to cause the interior threads on the endpiece **515** to be separated from the exterior threads on the body **505** to disconnect the endpiece **515** from the body **505,** thereby providing access to the chamber **115** so that the user may insert a capsule containing a pharmaceutical formulation. After insertion, the endpiece **515** is connected to the body by threaded engagement and the puncturing mechanism **525** is advanced to create one or more openings into the capsule. The user then places his or her mouth or nose on the endpiece **515** and inhales through the endpiece **515.** The inhalation causes air to flow through the inlets **510** and into the chamber **115** where it causes the capsule to be swirled in a manner which causes the pharmaceutical formulation to be aerosolized. The aerosolized pharmaceutical formulation then flows through the endpiece and into the user's respiratory tract. The twist attachment of the endpiece **515** to the body **505** prevents the inadvertent disconnection of the endpiece **515** as a result of inhalation pressure and thereby reduces the risk of inhalation of the endpiece **515.**

In a preferred version, the invention provides a system and method for aerosolizing a pharmaceutical formulation and delivering the pharmaceutical formulation to the lungs of the user. The pharmaceutical formulation may comprise powdered medicaments, liquid solutions or suspensions, and the like, and may include an active agent.

The active agent described herein includes an agent, drug, compound, composition of matter or mixture thereof which provides some pharmacologic, often beneficial, effect. This includes foods, food supplements, nutrients, drugs, vaccines, vitamins, and other beneficial agents. As used herein, the terms further include any physiologically or pharmacologically active substance that produces a localized or systemic effect in a patient. An active agent for incorporation in the pharmaceutical formulation described herein may be an inorganic or an organic compound, including, without limitation, drugs which act on: the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system, and the central nervous system. Suitable active agents may be selected from, for example, hypnotics and sedatives, psychic energizers, tranquilizers, respiratory drugs, anticonvulsants, muscle relaxants, antiparkinson agents (dopamine antagnonists), analgesics, anti-inflammatories, antianxiety drugs (anxiolytics), appetite suppressants, antimigraine agents, muscle contractants, anti-infectives (antibiotics, antivirals, antifungals, vaccines) antiarthritics, antimalarials, antiemetics, anepileptics, bronchodilators, cytokines, growth factors, anti-cancer agents, antithrombotic agents, antihypertensives, cardiovascular drugs, antiarrhythmics, antioxicants, anti-asthma agents, hormonal agents including contraceptives, sympathomimetics, diuretics, lipid regulating agents, antiandrogenic agents, antiparasitics, anticoagulants, neoplastics, antineoplastics, hypoglycemics, nutritional agents and supplements, growth supplements, antienteritis agents, vaccines, antibodies, diagnostic agents, and contrasting agents. The active agent, when administered by inhalation, may act locally or systemically.

The active agent may fall into one of a number of structural classes, including but not limited to small molecules, peptides, polypeptides, proteins, polysaccharides, steroids, proteins capable of eliciting physiological effects, nucleotides, oligonucleotides, polynucleotides, fats, electrolytes, and the like.

Examples of active agents suitable for use in this invention include but are not limited to one or more of calcitonin, erythropoietin (EPO), Factor VIII, Factor IX, ceredase, cerezyme, cyclosporin, granulocyte colony stimulating factor (GCSF), thrombopoietin (TPO), alpha-1 proteinase inhibitor, elcatonin, granulocyte macrophage colony stimulating factor (GMCSF), growth hormone, human growth hormone (HGH), growth hormone releasing hormone (GHRH), heparin, low molecular weight heparin (LMWH), interferon alpha, interferon beta, interferon gamma, interleukin-1 receptor, interleukin-2, interleukin-1 receptor antagonist, interleukin-3, interleukin-4, interleukin-6, luteinizing hormone releasing hormone (LHRH), factor IX, insulin, pro-insulin, insulin analogues (e.g., mono-acylated insulin as described in U.S. Patent No. 5,922,675), amylin, C-peptide, somatostatin, somatostatin analogs including octreotide, vasopressin, follicle stimulating hormone (FSH), insulin-like growth factor (IGF), insulintropin, macrophage colony stimulating factor (M-CSF), nerve growth factor (NGF), tissue growth factors, keratinocyte growth factor (KGF), glial growth factor (GGF), tumor necrosis factor (TNF), endothelial growth factors, parathyroid hormone (PTH), glucagon-like peptide thymosin alpha 1, IIb/IIIa inhibitor, alpha-1 antitrypsin, phosphodiesterase (PDE) compounds, VLA-4 inhibitors, bisphosponates, respiratory syncytial virus antibody, cystic fibrosis transmembrane regulator (CFTR) gene, deoxyreibonuclease (Dnase), bactericidal/permeability increasing protein (BPI), anti-CMV antibody, 13-cis retinoic acid, macrolides such as erythromycin, oleandomycin, troleandomycin, roxithromycin, clarithromycin, davercin, azithromycin, flurithromycin, dirithromycin, josamycin, spiromycin, midecamycin, leucomycin, miocamycin, rokitamycin, andazithromycin, and swinolide A; fluoroquinolones such as ciprofloxacin, ofloxacin, levofloxacin, trovafloxacin, alatrofloxacin, moxifloxicin, norfloxacin, enoxacin, grepafloxacin, gatifloxacin, lomefloxacin, sparfloxacin, temafloxacin, pefloxacin, amifloxacin, fleroxacin, tosufloxacin, prulifloxacin, irloxacin, pazufloxacin, clinafloxacin, and sitafloxacin, aminoglycosides such as gentamicin, netilmicin, paramecin, tobramycin, amikacin, kanamycin, neomycin, and streptomycin, vancomycin, teicoplanin, rampolanin, mideplanin, colistin, daptomycin, gramicidin, colistimethate, polymixins such as polymixin B, capreomycin, bacitracin, penems; penicillins including penicllinase-sensitive agents like penicillin G, penicillin V, penicillinase-resistant agents like methicillin, oxacillin, cloxacillin, dicloxacillin, floxacillin, nafcillin; gram negative microorganism active agents like ampicillin, amoxicillin, and hetacillin, cillin, and galampicillin; antipseudomonal penicillins like carbenicillin, ticarcillin, azlocillin, mezlocillin, and piperacillin; cephalosporins like cefpodoxime, cefprozil, ceftbuten, ceftizoxime, ceftriaxone, cephalothin, cephapirin, cephalexin, cephradrine, cefoxitin, cefamandole, cefazolin, cephaloridine, cefaclor, cefadroxil, cephaloglycin, cefuroxime, ceforanide, cefotaxime, cefatrizine, cephacetrile, cefepime, cefixime, cefonicid, cefoperazone, cefotetan, cefmetazole, ceftazidime, loracarbef, and moxalactam, monobactams like aztreonam; and carbapenems such as imipenem, meropenem, pentamidine isethiouate, albuterol sulfate, lidocaine, metaproterenol sulfate, beclomethasone diprepionate, triamcinolone acetamide, budesonide acetonide, fluticasone, ipratropium bromide, flunisolide, cromolyn sodium, ergotamine tartrate and where applicable, analogues, agonists, antagonists, inhibitors, and pharmaceutically acceptable salt forms of the above. In reference to peptides and proteins, the invention is intended to encompass synthetic, native, glycosylated, unglycosylated, pegylated forms, and biologically active fragments and analogs thereof.

Active agents for use in the invention further include nucleic acids, as bare nucleic acid molecules, vectors, associated viral particles, plasmid DNA or RNA or other nucleic acid constructions of a type suitable for transfection or transformation of cells, i.e., suitable for gene therapy including antisense. Further, an active agent may comprise live attenuated or killed viruses suitable for use as vaccines. Other useful drugs include those listed within the Physician's Desk Reference (most recent edition).

The amount of active agent in the pharmaceutical formulation will be that amount necessary to deliver a therapeutically effective amount of the active agent per unit dose to achieve the desired result. In practice, this will vary widely depending upon the particular agent, its activity, the severity of the condition to be treated, the patient population, dosing requirements, and the desired therapeutic effect. The composition will generally contain anywhere from about 1% by weight to about 99% by weight active agent, typically from about 2% to about 95% by weight active agent, and more typically from about 5% to 85% by weight active agent, and will also depend upon the relative amounts of additives contained in the composition. The compositions of the invention are particularly useful for active agents that are delivered in doses of from 0.001 mg/day to 100 mg/day, preferably in doses from 0.01 mg/day to 75 mg/day, and more preferably in doses from 0.10 mg/day to 50 mg/day. It is to be understood that more than one active agent may be incorporated into the formulations described herein and that the use of the term "agent" in no way excludes the use of two or more such agents.

The pharmaceutical formulation may comprise a pharmaceutically acceptable excipient or carrier which may be taken into the lungs with no significant adverse toxicological effects to the subject, and particularly to the lungs of the subject. In addition to the active agent, a pharmaceutical formulation may optionally include one or more pharmaceutical excipients which are suitable for pulmonary administration. These excipients, if present, are generally present in the composition in amounts ranging from about 0.01 % to about 95% percent by weight, preferably from about 0.5 to about 80%, and more preferably from about 1 to about 60% by weight. Preferably, such excipients will, in part, serve to further improve the features of the active agent composition, for example by providing more efficient and reproducible delivery of the active agent, improving the handling characteristics of powders, such as flowability and consistency, and/or facilitating manufacturing and filling of unit dosage forms. In particular, excipient materials can often function to further improve the physical and chemical stability of the active agent, minimize the residual moisture content and hinder moisture uptake, and to enhance particle size, degree of aggregation; particle surface properties, such as rugosity, ease of inhalation, and the targeting of particles to the lung. One or more excipients may also be provided to serve as bulking agents when it is desired to reduce the concentration of active agent in the formulation.

Pharmaceutical excipients and additives useful in the present pharmaceutical formulation include but are not limited to amino acids, peptides, proteins, non-biological polymers, biological polymers, carbohydrates, such as sugars, derivatized sugars such as alditols, aldonic acids, esterified sugars, and sugar polymers, which may be present singly or in combination. Suitable excipients are those provided in WO 96/32096. The excipient may have a glass transition temperatures (Tg) above about 35° C, preferably above about 40 °C, more preferably above 45° C, most preferably above about 55 °C.

Exemplary protein excipients include albumins such as human serum albumin (HSA), recombinant human albumin (rHA), gelatin, casein, hemoglobin, and the like. Suitable amino acids (outside of the dileucyl-peptides of the invention), which may also function in a buffering capacity, include alanine, glycine, arginine, betaine, histidine, glutamic acid, aspartic acid, cysteine, lysine, leucine, isoleucine, valine, methionine, phenylalanine, aspartame, tyrosine, tryptophan, and the like. Preferred are amino acids and polypeptides that function as dispersing agents. Amino acids falling into this category include hydrophobic amino acids such as leucine, valine, isoleucine, tryptophan, alanine, methionine, phenylalanine, tyrosine, histidine, and proline. Dispersibility- enhancing peptide excipients include dimers, trimers, tetramers, and pentamers comprising one or more hydrophobic amino acid components such as those described above.

Carbohydrate excipients suitable for use in the invention include, for example, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), pyranosyl sorbitol, myoinositol and the like.

The pharmaceutical formulation may also include a buffer or a pH adjusting agent, typically a salt prepared from an organic acid or base. Representative buffers include organic acid salts of citric acid, ascorbic acid, gluconic acid, carbonic acid, tartaric acid, succinic acid, acetic acid, or phthalic acid, Tris, tromethamine hydrochloride, or phosphate buffers.

The pharmaceutical formulation may also include polymeric excipients/additives, e.g., polyvinylpyrrolidones, derivatized celluloses such as hydroxymethylcellulose, hydroxyethylcellulose, and hydroxypropylmethylcellulose, Ficolls (a polymeric sugar), hydroxyethylstarch, dextrates (e.g., cyclodextrins, such as 2-hydroxypropyl-β-cyclodextrin and sulfobutylether-β-cyclodextrin), polyethylene glycols, and pectin.

The pharmaceutical formulation may further include flavoring agents, taste-masking agents, inorganic salts (for example sodium chloride), antimicrobial agents (for example benzalkonium chloride), sweeteners, antioxidants, antistatic agents, surfactants (for example polysorbates such as "TWEEN 20" and "TWEEN 80"), sorbitan esters, lipids (for example phospholipids such as lecithin and other phosphatidylcholines, phosphatidylethanolamines), fatty acids and fatty esters, steroids (for example cholesterol), and chelating agents (for example EDTA, zinc and other such suitable cations). Other pharmaceutical excipients and/or additives suitable for use in the compositions according to the invention are listed in "Remington: The Science & Practice of Pharmacy", 19th ed., Williams & Williams, (1995), and in the "Physician's Desk Reference", 52nd ed., Medical Economics, Montvale, NJ (1998).

"Mass median diameter" or "MMD" is a measure of mean particle size, since the powders of the invention are generally polydisperse (i.e., consist of a range of particle sizes). MMD values as reported herein are determined by centrifugal sedimentation, although any number of commonly employed techniques can be used for measuring mean particle size. "Mass median aerodynamic diameter" or "MMAD" is a measure of the aerodynamic size of a dispersed particle. The aerodynamic diameter is used to describe an aerosolized powder in terms of its settling behavior, and is the diameter of a unit density sphere having the same settling velocity, generally in air, as the particle. The aerodynamic diameter encompasses particle shape, density and physical size of a particle. As used herein, MMAD refers to the midpoint or median of the aerodynamic particle size distribution of an aerosolized powder determined by cascade impaction.

In one version, the powdered formulation for use in the present invention includes a dry powder having a particle size selected to permit penetration into the alveoli of the lungs, that is, preferably 10 µm mass median diameter (MMD), preferably less than 7.5 µm, and most preferably less than 5 µm, and usually being in the range of 0.1 µm to 5 µm in diameter. The delivered dose efficiency (DDE) of these powders may be greater than 30%, more preferably greater than 40%, more preferably greater than 50% and most preferably greater than 60% and the aerosol particle size distribution is about 1.0 - 5.0 µm mass median aerodynamic diameter (MMAD), usually 1.5 - 4.5 µm MMAD and preferably 1.5 - 4.0 µm MMAD. These dry powders have a moisture content below about 10% by weight, usually below about 5% by weight, and preferably below about 3% by weight. Such powders are described in WO 95/24183, WO 96/32149, WO 99/16419, and WO 99/16422.

Although the present invention has been described in considerable detail with regard to certain preferred versions thereof, other versions are possible, and alterations, permutations and equivalents of the version shown will become apparent to those skilled in the art upon a reading of the specification and study of the drawings. For example, the cooperating components may be reversed or provided in additional or fewer number. Also, the various features of the versions herein can be combined in various ways to provide additional versions of the present invention. Furthermore, certain terminology has been used for the purposes of descriptive clarity, and not to limit the present invention. Therefore, the appended claims should not be limited to the description of the preferred versions contained herein and should include all such alterations, permutations, and equivalents as fall within the scope of the present invention.

## Claims

1. An aerosolization apparatus (100) comprising:
a body (105) having an inlet (130); and
an endpiece (110) having an outlet (125), the endpiece (110) being connectable to the body (105) to define a chamber (115), wherein the chamber is sized to receive a capsule (160) containing a pharmaceutical formulation in a manner which allows the capsule to move within the chamber
**characterized by**:
the inlet (115) being shaped to provide a swirling air flow in the chamber;
a connection mechanism (150) to provide selective connection of the endpiece (110) to the body (105), wherein a rotational force between the endpiece and the body is needed to connect and to disconnect the endpiece from the body, the rotational force being applied about an axis passing through the chamber, and wherein the endpiece may be disconnected from the body to allow for insertion or removal of the capsule; and
a puncturing mechanism (162) capable of providing an opening in the capsule;
whereby when a user inhales, air enters into the chamber through the inlet so that the pharmaceutical formulation is aerosolized within the chamber and the aerosolized pharmaceutical formulation is delivered to the user through the outlet.

2. An aerosolization apparatus according to claim 1 wherein the body has a plurality of inlets.

3. An aerosolization apparatus according to claim 1 or 2 wherein the connection mechanism comprises engageable threads (310, 315).

4. An aerosolization apparatus according to any one of claims 1 to 3 wherein the connection mechanism comprises a protrusion (175) that is receivable within a slot (180), the slot comprising a longitudinally extending portion (185) and a transversely extending portion (190).

5. An aerosolization apparatus according to any one of claims 1 to 4, wherein the puncturing mechanism is adapted to puncture a single end of the capsule.

6. An apparatus according to any one of claims 1 to 5, wherein the chamber is elongated and has a longitudinal axis, and wherein the longitudinal axis of the chamber and the longitudinal axis of the capsule form an angle of less than about 45 degrees during use.

7. An apparatus according to any one of claims 1 to 6 wherein the chamber is elongated and wherein the capsule is received lengthwise within the elongated chamber.

8. An aerosolization apparatus according to one of claims 1 to 7 wherein the width of the chamber is less than the length of the capsule.

9. An aerosolization apparatus (100) comprising:
a body (105) having an inlet (130);
an endpiece (110) having an outlet (125), the endpiece being connectable to the body to define a chamber (115), wherein the chamber is sized to receive a capsule (160) containing a pharmaceutical formulation in a manner which allows the capsule to move within the chamber,
a connection mechanism (150) to provide selective connection of the endpiece to the body, wherein the connection mechanism comprises engageable threads and the endpiece may be disconnected from the body to allow for insertion or removal of the capsule;
**characterized by**:
the inlet being shaped to provide a swirling air flow (170) in the chamber; and
a puncturing mechanism (162) capable of providing an opening in the capsule:
whereby when a user inhales, air enters into the chamber through the inlet so that the pharmaceutical formulation is aerosolized within the chamber and the aerosolized pharmaceutical formulation is delivered to the user through the outlet.

10. An aerosolization apparatus according to any one of claims 3 to 9 wherein the connection mechanism comprises engageable threads (310, 315) having a thread angle of less than about 9 degrees.

11. An aerosolization apparatus according to any one of claims 3 to 10
wherein the engageable threads include a recessed portion (320).

12. An aerosolization apparatus according to claim 11 wherein the recessed portion is recessed at an angle of less than about 75 degrees.

13. An aerosolization apparatus comprising:
a body having an inlet; and
an endpiece having an outlet, the endpiece being connectable to the body to define a chamber, wherein the chamber is sized to receive a capsule containing a pharmaceutical formulation in a manner which allows the capsule to move within the chamber
**characterized by**:
the inlet being shaped to provide a swirling air flow in the chamber;
a connection mechanism to provide selective connection of the endpiece to the body, wherein the connection mechanism comprises a protrusion that is receivable within a slot, the slot comprising a longitudinally extending portion and a transversely extending portion, and wherein the endpiece may be connected to the body by sliding the protrusion in the transversely extending portion in a first direction and wherein the endpiece may be disconnected from the body by sliding the protrusion in the transversely extending portion in an opposite direction to allow for insertion or removal of the capsule; and
a puncturing mechanism capable of providing an opening in the capsule;
whereby during inhalation air enters into the chamber through the inlet so that the pharmaceutical formulation is aerosolized within the chamber and the aerosolized pharmaceutical formulation is delivered to the user through the outlet.

14. An aerosolization apparatus according to claim 13 wherein the slot further comprises means for securing the protrusion (175) within the slot (180).

15. An aerosolization apparatus according to claim 14 wherein the means for securing comprises a bump (195) on the transversely extending portion (190).

16. An aerosolization apparatus according to claims 14 or 15 wherein the means for securing comprises one or more sloping surfaces in the slot (180).

17. An aerosolization apparatus according to claims 14, 15 or 16 wherein the means for securing comprises a second longitudinally extending portion (185) of the slot (180).

## Patentansprüche

1. Aerosolisiervorrichtung (100) mit:
einem Körper (105) mit einem Einlass (130); und
einem Endstück (110) mit einem Auslass (125), wobei das Endstück (110) mit dem Körper (105) verbindbar ist, um einen Raum (115) zu definieren, wobei der Raum entsprechend bemessen ist, um eine Kapsel (160), die eine pharmazeutische Formulierung enthält, aufzunehmen, so dass die Kapsel sich innerhalb des Raumes bewegen kann;
**dadurch gekennzeichnet dass**:
der Einlass (115) so geformt ist, dass ein Luftwirbelstrom in dem Raum entsteht; und mit
einem Verbindungsmechanismus (150) zur selektiven Verbindung des Endstücks (110) mit dem Körper (105), wobei eine Drehkraft zwischen dem Endstück und dem Körper benötigt wird, um das Endstück mit dem Körper zu verbinden und von diesem zu trennen, wobei die Drehkraft um eine Achse, die durch den Raum verläuft, ausgeübt wird und wobei das Endstück vom Körper getrennt werden kann, um Einsetzung oder Entnahme der Kapsel zu ermöglichen; und
einem Einstichmechanismus (162), um eine Öffnung in die Kapsel einzubringen;
wodurch, wenn ein Benutzer inhaliert, Luft durch den Einlass in den Raum eintritt, so dass die pharmazeutische Formulierung innerhalb des Raumes aerosolisiert und die aerosolisierte pharmazeutische Formulierung durch den Auslass an den Benutzer abgegeben wird.

2. Aerosolisiervorrichtung nach Anspruch 1, wobei der Körper mehrere Einlässe hat.

3. Aerosolisiervorrichtung nach Anspruch 1 oder 2, wobei der Verbindungsmechanismus eingriffsfähige Gewinde (310, 315) aufweist.

4. Aerosolisiervorrichtung nach einem der Ansprüche 1 bis 3, wobei der Verbindungsmechanismus einen Vorsprung (175) aufweist, der in einem Schlitz (180) aufgenommen werden kann, wobei der Schlitz einen sich längs erstreckenden Abschnitt (185) und einen sich quer erstreckenden Abschnitt (190) hat.

5. Aerosolisiervorrichtung nach einem der Ansprüche 1 bis 4, wobei der Einstichmechanismus entsprechend angepasst ist, um ein einziges Ende der Kapsel zu durchstechen.

6. Vorrichtung nach einem der Ansprüche 1 bis 5, wobei der Raum langgestreckt ist und eine Längsachse hat und wobei die Längsachse des Raums und die Längsachse der Kapsel bei Benutzung einen Winkel bilden, der kleiner als etwa 45 Grad ist.

7. Vorrichtung nach einem der Ansprüche 1 bis 6, wobei der Raum langgestreckt ist und wobei die Kapsel der Länge nach in dem langgestreckten Raum aufgenommen wird.

8. Aerosolisiervorrichtung nach einem der Ansprüche 1 bis 7, wobei Breite des Raumes kleiner als die Länge der Kapsel ist.

9. Aerosolisiervorrichtung (100) mit:
einem Körper (105) mit einem Einlass (130);
einem Endstück (110) mit einem Auslass (125), wobei das Endstück mit dem Körper verbindbar ist, um einen Raum (115) zu definieren, wobei der Raum entsprechend bemessen ist, um eine Kapsel (160), die eine pharmazeutische Formulierung enthält, aufzunehmen, so dass die Kapsel sich innerhalb des Raumes bewegen kann; und
einem Verbindungsmechanismus (150) zur selektiven Verbindung des Endstücks mit dem Körper, wobei der Verbindungsmechanismus eingriffsfähige Gewinde aufweist und das Endstück von dem Körper getrennt werden kann, um Einsetzung oder Entnahme der Kapsel zu ermöglichen;
**dadurch gekennzeichnet dass**:
der Einlass so geformt ist, dass ein Luftwirbelstrom (170) in dem Raum entsteht; und mit
einem Einstichmechanismus (162) mit der Fähigkeit, eine Öffnung in die Kapsel einzubringen;
wodurch, wenn ein Benutzer inhaliert, Luft durch den Einlass in den Raum eintritt, so dass die pharmazeutische Formulierung innerhalb des Raumes aerosolisiert und die aerosolisierte pharmazeutische Formulierung durch den Auslass an den Benutzer abgegeben wird.

10. Aerosolisiervorrichtung nach einem der Ansprüche 3 bis 9, wobei der Verbindungsmechanismus eingriffsfähige Gewinde (310, 315) mit einem Flankenwinkel aufweist, der kleiner als etwa 9 Grad ist.

11. Aerosolisiervorrichtung nach einem der Ansprüche 3 bis 10, wobei die eingriffsfähigen Gewinde einen ausgesparten Abschnitt (320) aufweisen.

12. Aerosolisiervorrichtung nach Anspruch 11, wobei der ausgesparte Abschnitt mit einem Winkel von kleiner als etwa 75 Grad ausgespart ist.

13. Aerosolisiervorrichtung mit:
einem Körper mit einem Einlass; und
einem Endstück mit einem Auslass, wobei das Endstück mit dem Körper verbunden werden kann, um einen Raum zu definieren, wobei der Raum entsprechend bemessen ist, um eine Kapsel, die eine pharmazeutische Formulierung enthält, derartig aufzunehmen, dass die Kapsel sich innerhalb des Raumes bewegen kann,
**dadurch gekennzeichnet dass**:
der Einlass so geformt ist, dass ein Luftwirbelstrom in dem Raum entsteht; und mit
einem Verbindungsmechanismus zur selektiven Verbindung des Endstücks mit dem Körper, wobei der Verbindungsmechanismus einen Vorsprung aufweist, der in einem Schlitz aufgenommen werden kann, wobei der Schlitz einen sich längs erstreckenden Abschnitt und einen sich quer erstreckenden Abschnitt aufweist und wobei das Endstück mit dem Körper verbunden werden kann, indem der Vorsprung in dem sich längs erstreckenden Abschnitt in einer ersten Richtung verschoben wird, und wobei das Endstück vom Körper getrennt werden kann, indem der Vorsprung in dem sich quer erstreckenden Abschnitt in einer entgegengesetzten Richtung verschoben wird, um Einsetzen und Entnahme der Kapsel zu ermöglichen; und
einem Einstichmechanismus, um eine Öffnung in die Kapsel einzubringen;
wodurch während der Inhalation Luft durch den Einlass in den Raum eintritt, so dass die pharmazeutische Formulierung innerhalb des Raumes aerosolisiert und die aerosolisierte pharmazeutische Formulierung durch den Auslass an den Benutzer abgegeben wird.

14. Aerosolisiervorrichtung nach Anspruch 13, wobei der Schlitz ferner eine Sicherungseinrichtung (175) des Vorsprungs in dem Schlitz (180) aufweist.

15. Aerosolisiervorrichtung nach Anspruch 14, wobei die Sicherungseinrichtung eine Erhöhung (195) auf dem sich quer erstreckenden Abschnitt (190) aufweist.

16. Aerosolisiervorrichtung nach Anspruch 14 oder 15, wobei die Sicherungseinrichtung eine oder mehrere geneigte Flächen im Schlitz (180) aufweist.

17. Aerosolisiervorrichtung nach einem der Ansprüche 14, 15 oder 16, wobei die Sicherungseinrichtung einen zweiten sich längs erstreckenden Abschnitt (185) des Schlitzes (180) aufweist.

## Revendications

1. Appareil de pulvérisation en aérosol (100) comprenant :
► un corps (105) ayant une entrée (130) ; et
► une pièce d'extrémité (110) ayant une sortie (125), la pièce d'extrémité (110) pouvant être raccordée au corps (105) pour définir une chambre (115), où la chambre est dimensionnée pour recevoir une capsule (160) contenant une formulation pharmaceutique d'une manière qui permet à la capsule de se déplacer dans la chambre,
**caractérisé par** :
► l'entrée (115) ayant une forme permettant de former un écoulement d'air tourbillonnant dans la chambre ;
► un mécanisme de raccordement (150) pour assurer un raccordement sélectif de la pièce d'extrémité (110) au corps (105), où une force de rotation entre la pièce d'extrémité et le corps est nécessaire pour raccorder la pièce d'extrémité au corps et la découpler de celui-ci, la force de rotation étant appliquée autour d'un axe traversant la chambre, et où la pièce d'extrémité peut être découplée du corps pour permettre l'insertion ou le retrait de la capsule ; et
► un mécanisme de perforation (162) capable de ménager une ouverture dans la capsule ;
moyennant quoi lorsqu'un utilisateur inhale, l'air entre dans la chambre à travers l'entrée de sorte que la formulation pharmaceutique est pulvérisée en aérosol dans la chambre et la formulation pharmaceutique pulvérisée en aérosol est délivrée à l'utilisateur par la sortie.

2. Appareil de pulvérisation en aérosol selon la revendication 1, dans lequel le corps comporte une pluralité d'entrées.

3. Appareil de pulvérisation en aérosol selon la revendication 1 ou 2, dans lequel le mécanisme de raccordement comprend des filetages pouvant être mis en prise (310, 315).

4. Appareil de pulvérisation en aérosol selon l'une quelconque des revendications 1 à 3, dans lequel le mécanisme de raccordement comprend une saillie (175) qui peut être reçue dans une fente (180), la fente comprenant une portion en extension longitudinale (185) et une portion en extension transversale (190).

5. Appareil de pulvérisation en aérosol selon l'une quelconque des revendications 1 à 4, dans lequel le mécanisme de perforation est adapté pour perforer une seule extrémité de la capsule.

6. Appareil selon l'une quelconque des revendications 1 à 5, dans lequel la chambre est allongée et présente un axe longitudinal, et dans lequel l'axe longitudinal de la chambre et l'axe longitudinal de la capsule forment un angle inférieur à environ 45 degrés en utilisation.

7. Appareil selon l'une quelconque des revendications 1 à 6, dans lequel la chambre est allongée et dans lequel la capsule est reçue dans le sens de la longueur dans la chambre allongée.

8. Appareil de pulvérisation en aérosol selon l'une quelconque des revendications 1 à 7, dans lequel la largeur de la chambre est inférieure à la longueur de la capsule.

9. Appareil de pulvérisation en aérosol (100) comprenant :
► un corps (105) ayant une entrée (130) ;
► une pièce d'extrémité (110) ayant une sortie (125), la pièce d'extrémité pouvant être raccordée au corps pour définir une chambre (115), où la chambre est dimensionnée pour recevoir une capsule (160) contenant une formulation pharmaceutique d'une manière qui permet à la capsule de se déplacer dans la chambre, un mécanisme de raccordement (150) pour assurer un raccordement sélectif de la pièce d'extrémité au corps, où le mécanisme de raccordement comprend des filetages pouvant être mis en prise et la pièce d'extrémité peut être découplée du corps pour permettre l'insertion ou le retrait de la capsule ;
**caractérisé par** :
► l'entrée ayant une forme permettant de former un écoulement d'air tourbillonnant (170) dans la chambre ; et
► un mécanisme de perforation (162) capable de ménager une ouverture dans la capsule ;
moyennant quoi lorsqu'un utilisateur inhale, l'air entre dans la chambre à travers l'entrée de sorte que la formulation pharmaceutique est pulvérisée en aérosol dans la chambre et la formulation pharmaceutique pulvérisée en aérosol est délivrée à l'utilisateur par la sortie.

10. Appareil de pulvérisation en aérosol selon l'une quelconque des revendications 3 à 9, dans lequel le mécanisme de raccordement comprend des filetages pouvant être mis en prise (310, 315) ayant un angle de filet inférieur à environ 9 degrés.

11. Appareil de pulvérisation en aérosol selon l'une quelconque des revendications 3 à 10, dans lequel les filetages pouvant être mis en prise incluent une portion évidée (320).

12. Appareil de pulvérisation en aérosol selon la revendication 11, dans lequel la portion évidée est évidée selon un angle inférieur à environ 75 degrés.

13. Appareil de pulvérisation en aérosol comprenant :
► un corps ayant une entrée ; et
► une pièce d'extrémité ayant une sortie, la pièce d'extrémité pouvant être raccordée au corps pour définir une chambre, où la chambre est dimensionnée pour recevoir une capsule contenant une formulation pharmaceutique d'une manière qui permet à la capsule de se déplacer dans la chambre,
**caractérisé par** :
► l'entrée ayant une forme permettant de former un écoulement d'air tourbillonnant dans la chambre ;
► un mécanisme de raccordement pour assurer un raccordement sélectif de la pièce d'extrémité au corps, où le mécanisme de raccordement comprend une saillie qui peut être reçue dans une fente, la fente comprenant une portion en extension longitudinale et une portion en extension transversale, et où la pièce d'extrémité peut être raccordée au corps en faisant glisser la saillie dans la portion en extension transversale dans une première direction et où la pièce d'extrémité peut être découplée du corps en faisant glisser la saillie dans la portion en extension transversale dans une direction opposée pour permettre l'insertion ou le retrait de la capsule ; et
► un mécanisme de perforation capable de ménager une ouverture dans la capsule ;
moyennant quoi pendant l'inhalation, de l'air entre dans la chambre par l'entrée de sorte que la formulation pharmaceutique est pulvérisée en aérosol dans la chambre et la formulation pharmaceutique pulvérisée en aérosol est délivrée à l'utilisateur par la sortie.

14. Appareil de pulvérisation en aérosol selon la revendication 13, dans lequel la fente comprend en outre un moyen pour arrimer la saillie (175) dans la fente (180).

15. Appareil de pulvérisation en aérosol selon la revendication 14, dans lequel le moyen d'arrimage comprend une bosse (195) sur la portion en extension transversale (190).

16. Appareil de pulvérisation en aérosol selon la revendication 14 ou 15, dans lequel le moyen d'arrimage comprend une ou plusieurs surfaces inclinées dans la fente (180).

17. Appareil de pulvérisation en aérosol selon la revendication 14, 15 ou 16, dans lequel le moyen d'arrimage comprend une deuxième portion en extension longitudinale (185) de la fente (180).
